# EUROPEAN PATENT SPECIFICATION

(11) **EP 3 207 926 B1**
(45) Date of publication and mention of the grant of the patent: **31.03.2021**
(21) Application number: 16156194.9
(22) Date of filing: 17.02.2016
(51) Int. Cl.: A61K 31/201, A61K 31/336, A61K 31/4184, A61K 31/445, A61K 31/496, A61P 25/02, G01N 33/68

(54) **OXIDIZED LIPIDS IN THE TREATMENT OF CHRONIC OR NEUROPATHIC PAIN**
OXIDIERTE LIPIDE ZUR BEHANDLUNG BEI CHRONISCHEN ODER NEUROPATHISCHEN SCHMERZEN
LIPIDES OXYDÉS DANS LE TRAITEMENT DE LA DOULEUR NEUROPATHIQUE CHRONIQUE

(43) Date of publication of application: 23.08.2017
(73) Proprietor: Fraunhofer-Gesellschaft zur Förderung der angewandten Forschung e.V., 80686 München (DE); Goethe-Universität Frankfurt am Main, 60323 Frankfurt am Main (DE)
(72) Inventor: SISSIGNANO, Marco, 60385 Frankfurt (DE); GEISSLINGER, Gerd, 65812 Bad Soden (DE); PARNHAM, Michael John, 65812 Bad Soden/Ts (DE)
(74) Representative: Altmann Stößel Dick Patentanwälte PartG mbB

(56) References cited:
- EP-A1- 2 642 293
- WO-A1-2009/062073
- WO-A1-2016/024015
- WO-A2-2010/062900
- B. INCEOGLU ET AL: "Acute augmentation of epoxygenated fatty acid levels rapidly reduces pain-related behavior in a rat model of type I diabetes", PROCEEDINGS OF THE NATIONAL ACADEMY OF SCIENCES, vol. 109, no. 28, 25 June 2012 (2012-06-25), pages 11390-11395, XP055291837, US ISSN: 0027-8424, DOI: 10.1073/pnas.1208708109
- SHUNJI KUNORI ET AL: "A novel role of prostaglandin E2 in neuropathic pain", GLIA, vol. 59, no. 2, 1 February 2011 (2011-02-01), pages 208-218, XP055291731, US ISSN: 0894-1491, DOI: 10.1002/glia.21090
- RAMSDEN CHRISTOPHER E ET AL: "Targeted alteration of dietary n-3 and n-6 fatty acids for the treatment of chronic headaches: A randomized trial", PAIN, vol. 154, no. 11, 1 November 2013 (2013-11-01), pages 2441-2451, XP028744054, ISSN: 0304-3959, DOI: 10.1016/J.PAIN.2013.07.028
- POULSEN J N ET AL: "IBUDILAST AND SKF MODULATE CISPLATIN-EVOKED PGE2 RELEASE FROM ISOLATED TRIGEMINAL SATELLITE GLIAL CELLS", GLIA, vol. 61, no. Suppl. 1, July 2013 (2013-07) , page S109, XP002760364, & 11TH EUROPEAN MEETING ON GLIAL CELL FUNCTION IN HEALTH AND DISEASE; BERLIN, GERMANY; JULY 03 -06, 2013
- ANU UPADHYAY: "Reflex Sympathetic Dystrophy-Like Syndrome, Possibly Caused By Aripiprazole, in an Adolescent Patient", JOURNAL OF CHILD AND ADOLESCENT PSYCHOPHARMACOLOGY, vol. 24, no. 7, 1 September 2014 (2014-09-01), pages 414-415, XP55220115, US ISSN: 1044-5463, DOI: 10.1089/cap.2014.0032
- Amol M. Patwardhan ET AL: "Heat generates oxidized linoleic acid metabolites that activate TRPV1 and produce pain in rodents", Journal of Clinical Investigation, vol. 120, no. 5, 3 May 2010 (2010-05-03), pages 1617-1626, XP55042034, ISSN: 0021-9738, DOI: 10.1172/JCI41678

## Description

### Field of the invention

The present invention pertains to the use of oxidized lipids as biomarkers in the determination and treatment of chronic or neuropathic pain; in particular chemotherapy induced peripheral neuropathic pain (CIPNP).

### Description

Neuropathic pain is a persistent or chronic pain syndrome that can result from damage to the nervous system, the peripheral nerves, the dorsal root ganglion, dorsal root, or to the central nervous system. Neuropathic pain syndromes include allodynia, various neuralgias such as post herpetic neuralgia and trigeminal neuralgia, phantom pain, and complex regional pain syndromes, such as reflex sympathetic dystrophy and causalgia. Causalgia is often characterized by spontaneous burning pain combined with hyperalgesia and allodynia. Tragically there is no existing method for adequately, predictably and specifically treating established neuropathic pain as present treatment methods for neuropathic pain consist of merely trying to help the patient cope through psychological or occupational therapy, rather than by reducing or eliminating the pain experienced. Treatment of neuropathic or chronic pain is a challenge for physicians and patients since there are no medications that specifically target the condition, and since the medications presently used result in only little relief and are based on their efficacy in acute pain conditions or on their efficacy on relieving secondary effects like anxiety and depression. Incidence of chronic pain is increasing in society and its burden on society is huge in both health care and lost productivity. Currently there are no scientifically validated therapies for relieving chronic pain. As a result, the health community targets 'pain management' where multi-modal therapies are used concurrently with the hope of providing some improvement in quality of life. Thus, there is an urgent need for drugs that can relieve chronic pain.

Chemotherapy induced peripheral neuropathic pain (CIPNP) is a severe dose limiting side effect of cytostatics, such as taxanes, platinum derivates, vinca alkaloids and others. The symptoms usually start with tingling and can lead to burning, stabbing and aching pain as well as cold and mechanical allodynia. Due to CIPNP some patients stop anticancer therapy with cytostatics too early, resulting in a higher risk of tumor progression. Unfortunately, many promising substances that are already approved for the treatment of different kinds of neuropathic pain, such as gabapentin or amitriptyline seem to have little or no analgesic effect in monotherapy of CIPNP. Understanding the cellular and molecular mechanisms is necessary to treat or even prevent CIPNP and may improve the general success rate of cytostatic therapy.

Early therapeutic intervention is crucial for the therapy of neuropathic pain [1, 2]. For this reason, biomarkers are especially important for neuropathic pain and represent important diagnostic markers that may be used for therapeutic strategies. Particularly during treatment of patients with cytostatics or during diabetes, the onset and intensity of neuropathic pain varies strongly among patients. In the ideal case, biomarkers may be measured from plasma of patients and analyzed for their concentrations. This can be used to predict onset, intensity and duration of neuropathic pain even before the first symptoms arise in patients [3]. In this regard, high-risk patients could be treated preventatively with drugs that are effective for the treatment of neuropathic pain, such as amitriptyline, gabapentin or duloxetine as early as possibly to reduce or even prevent neuropathic pain.

Currently, there are no biomarkers available for the prediction of onset, intensity or duration of neuropathic pain in patients. However, there is a strong demand for such biomarkers because neuropathic pain is still difficult to treat and can persist lifelong, especially when it is already fully established. There are several studies concerning genetic causes [7], such as single nucleotide polymorphisms or punctual mutations, that may explain some pain syndromes (like the familial episodic pain syndrome [8]), however, to this day, there are no predictive biomarkers, that could be used to judge a person's susceptibility to developing neuropathic pain.

The above problem is solved in a first aspect by an oxidized lipid for use in the diagnosis, prevention or treatment of chronic or neuropathic pain, which is peripheral neuropathy due to chemotherapy.

The epoxylipid is selected from the group
consisting of: 9, 10-EpOME ((±)9(1 O)-epoxy-12Z-octadecaenoic acid), 9-HODE ((±)9-hydroxy-1O(E),12(Z)-octadecadienoic acid) and 13-HODE ((±)13-hydroxy-9(Z), 11 (E)-octadecadienoic acid), most preferably is 9,10-EpOME ((±)9(10)-epoxy-12Z-octadecaenoic acid).

Preferred embodiments of the present invention include:
The lipid for use in the diagnosis, prevention or treatment of neuropathic pain in a subject, wherein said neuropathic pain is chemotherapy-induced peripheral neuropathic pain (CIPNP).

The present invention also provides the use of the oxidized lipid as above stated as a biomarker in a method for the prediction of onset, intensity or duration of neuropathic pain in a subject.

In another embodiment of the present invention, the concentration of the oxidized lipid is measured after 24 hours after the start of chemotherapy.

In a further embodiment of the invention, the concentration of the oxidized lipid is measured from plasma. In another embodiment, the concentration is measured using LC-MS/MS.

According to the present invention, the treatment of the pain in a subject starts when the concentration of the epoxylipid and/or oxidized lipid is at least 20%, preferably 30%, more preferably 40% higher than the normal value.

Further provided by the present invention is a method for the prediction of onset, intensity or duration of neuropathic pain in a subject, comprising:
a) separating plasma from blood sample;
b) measuring the concentration of an oxidized lipid in the plasma;
c) determining the ratio of the elevation of the oxidized lipid.

The epoxylipid is selected from the group consisting of: 9.10-EpOME ((±)9(1O)-epoxy-12Z-octadecaenoic acid), 9-HODE ((±)9-hydroxy-1O(E),12(Z)-octadecadienoic acid) and 13-HODE ((±)13-hydroxy-9(Z), 11(E)-octadecadienoic acid), preferably is 9,10-EpOME ((±)9(1O)-epoxy-12Z-octadecaenoic acid).

The concentration of the oxidized lipid is measured as
early as 24 hours after the beginning of a chemo-therapy.

The present invention further provides a therapeutic treatment of
peripheral neuropathy due to chemotherapy ina subject, characterized in that the therapy starts before the first symptoms arise in patient.
The treatment of peripheral neuropathy due to chemotherapy encompasses a cytochrome P450 epoxygenase (CYP)-antagonist.
The therapy starts after the
elevation of an oxidized lipid has been detected according to the method of this invention.
In some embodiments of the invention the CYP-antagonist is selected from the group consisting of a CYP1A-, CYP2B-, CYP2G-, CYP2E-, and preferably a CYP2J-antagonist. Most preferably the CYP-antagonist is an antagonist of a mammalian homologue of CYP2J2 (CYP2J2-antagonist), preferably human CYP2J2, such as telmisartan, aripiprazole or most preferably terfenadine.

A further preferred therapeutic treatment is the use of any CYP2J2
antagonist, preferably a selective CYP2J2 antagonist. The term "selective CYP2J2 antagonist" pertains to antagonists of CYP2J2 that selectively inhibit activity, function or expression of CYP2J2 but not of other related enzymes such as for example CYP3A molecules. In order to identify whether a candidate antagonist is a CYP2J2 antagonist, a luminogenic cytochrome P450 glow assay can be employed. CYP proteins catalyse the formation of arachidonic acid metabolites. Luminogenic CYP assays use prosubstrates for the light-generating reaction of luciferase. CYPs convert the prosubstrates to luciferin or a luciferin ester, which produces light in a second reaction with a luciferase reaction mix called Luciferin Detection Reagent (LDR). The amount of light produced in the second reaction is proportional to CYP activity.

Another pain therapy comprising the inhibition of the activity of in particular CYP2J2 which produces the metabolic compound 9,10-EpOME - according to the invention, a sensitizer of ion channel-mediated pain perception. Surprisingly, the inhibition of CYP2J2 in accordance with the invention proved to be effective *in-vivo* to alleviate neuropathic pain induced by paclitaxel in a mouse model, indicating the use of CYP2J2 antagonists as analgesic against neuropathic pain, in particular CIPNP.

One further embodiment of the invention relates to the above mentioned prevention or treatment of pain, which comprises the administration of said CYP antagonist of the invention to a subject suffering from said pain, and wherein said subject received, receives or will receive chemotherapy. Therefore, the subject is in preferred embodiments a subject suffering from, or diagnosed with, a cancer disease.

Chemotherapy in context of the invention preferably involves the administration of a chemotherapeutic agent to a subject in need of such a treatment selected from pyrimidinone-based anti-neoplastic agents such as cytarabine, 5-flurouracil or platin agents, such as cisplatin, or taxanes, such as paclitaxel, docetaxel or cabazitaxel, or derivatives thereof. Such chemotherapeutic agents are known to induce neuropathic pain, in particular this is known for taxanes, which are therefore preferred in context of the invention. Most preferred is paclitaxel.

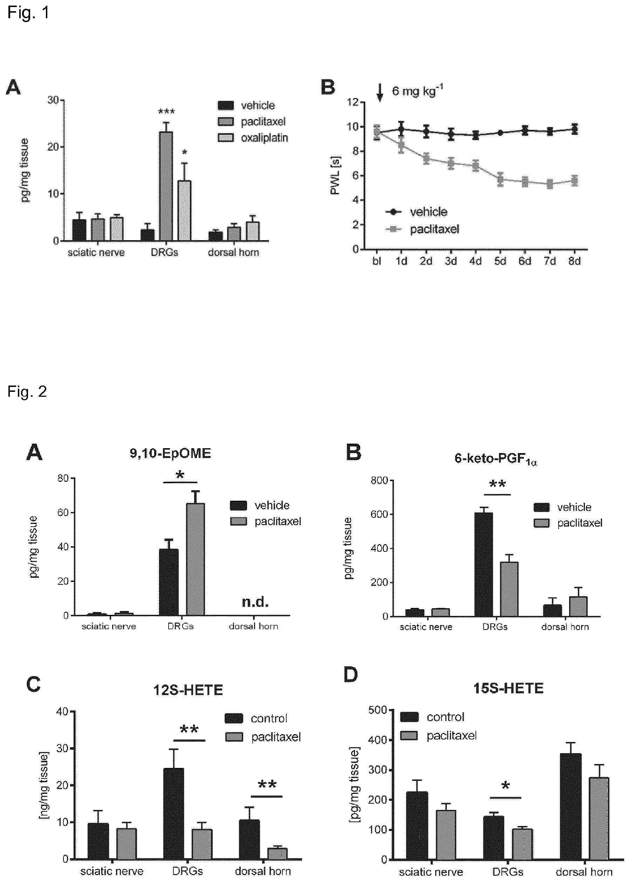

In animal models of neuropathic pain [4, 5], we use LC-MS/MS to perform lipid profiling and surprisingly observed increased concentrations of specific epoxylipids and oxidized lipids from the arachidonic and linoleic acid pathway. Particularly, the concentrations of 9,10-EpOME ((±)9(10)-epoxy-12Z-octadecaenoic acid), 9-HODE ((±)9-hydroxy-1O(*E*),12(*Z*)-octadecadienoic acid) and 13-HODE ((±)13-hydroxy-9(*Z*),11(*E*)-octadecadienoic acid) (Figure 1-3) are found to be elevated in various pain relevant tissues and in the plasma following paclitaxel- or oxaliplatin-treatment which leads to peripheral neuropathic pain. These increased concentrations can already be measured as early as 24 hours after paclitaxel or oxaliplatin-injection in mice, even before the animals develop pain (Figure 1). Usually, it takes around seven days until the peripheral neuropathy is developed in these models (Figure 1, right) [4-6]. Moreover, the concentrations of the arachidonic acid metabolites 12-HETE ((±)12-hydroxy-5Z,8Z,10E,14Z-eicosatetraenoic acid), 15-HETE ((±)15-hydroxy-5Z,8Z,11Z,13E-eicosatetraenoic acid) und 6-keto-Prostaglandin-F_{1α} are found to be decreased in pain relevant tissues (Figure 2). Taken together, these results imply a mass spectrometric based quantification of the ratio of 9,10-EpOME and other oxidized lipids in plasma as promising biomarkers for predicting increased risks for the development of neuropathic pain. If this is successful in patients, a physician could for example treat patients with increased 9,10-EpOME or 9-HODE concentrations in the plasma preventatively to reduce or even prevent the occurrence of neuropathic pain.

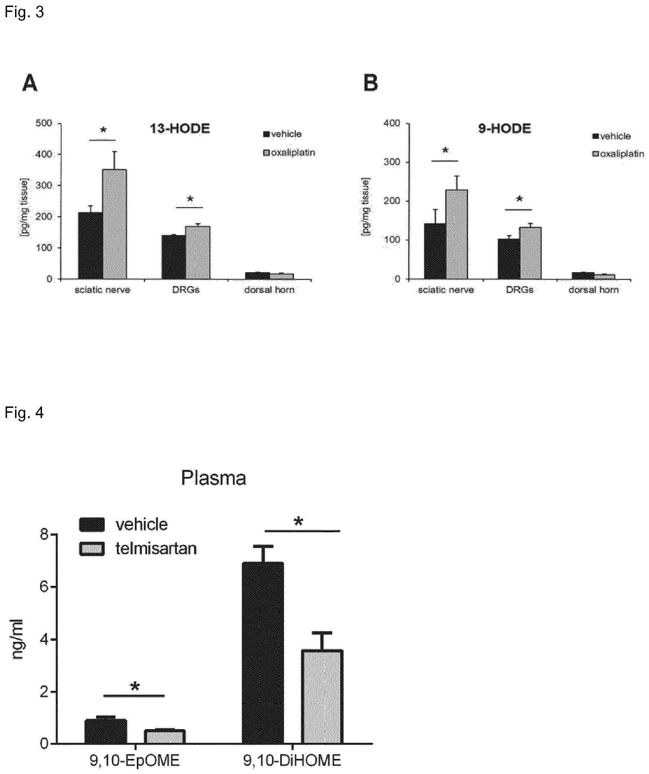

9,10-EpOME and the other oxidized lipids can be measured easily from plasma (Figure 4). Only a small volume of about 100 µl is needed for this analysis. Cancer patients that suffer from adverse events of chemotherapy or diabetes patients have to undergo blood analysis regularly, so that a small part of blood could be used for lipid anaylsis without additional stress or distraction. Measuring lipids with LC-MS/MS is a standard method (described below) that has already been established in many labs, so that the analysis of oxidized lipids from plasma can be performed rapidly, usually within a day after obtaining plasma from patients. Other methods to measure these biomarkers or their ratios, such as enzyme immune assays as a commercially available kit can also be used for the detection of these lipids.

**Table 1: Formal chemical classification of putative lipid biomarkers**

| **Abbreviated name** | **Formal name** | **InChIKey** | **Cas-number** |
|---|---|---|---|
| 9,10-EpOME | (±)9(10)-epoxy-12Z-octadecaenoic acid | FBUKMFOXMZRGRB-XKJZPFPASA-N | 6814-52-4 |
| 9,10-DiHOME | (±)9(10)-dihydroxy-12Z-octadecenoic acid | XEBKSQSGNGRGDW-CJWPDFJNSA-N | 263399-34-4 |
| 9-HODE | (±)-9-hydroxy-10E, 12Z-octadecadienoic acid | NPDSHTNEKLQQIJ-ZJHFMPGASA-N | 98524-19-7 |
| 13-HODE | (±)-13-hydroxy-9Z, 11 E-octadecadienoic acid | HNICUWMFWZBIFP-BSZOFBHHSA-N | 73804-64-5 |
| 12-HETE | (±)12-hydroxy-5Z,8Z,10E,14Z-eicosatetraenoic acid | ZNHVWPKMFKADKW-VXBMJZGYSA-N | 71030-37-0 |
| 15-HETE | (±)15-hydroxy-5Z,8Z,11Z,13E-eicosatetraenoic acid | JSFATNQSLKRBCI-USWFWKISSA-N | 73836-87-0 |
| 6-keto-PGF_{1α} | 6-oxo-9α,11α,15S-trihydroxy-prost-13E-en-1-oic acid | KFGOFTHODYBSGM-ZUNNJUQCSA-N | 58962-34-8 |

**Table 2: Group of oxidized lipids measured by LC-MS/MS contains:**

| **• 2a: COX-metabolites** | | | |
|---|---|---|---|
| **Abbreviated name** | **Formal name** | **InChIKey** | **Cas-number** |
| PGE2, Prostaglandin E₂ | 9-oxo-11.alpha.,15S-dihydroxy-prosta-5Z,13E-dien-1-oic acid | XEYBRNLFEZDVAW-ARSRFYASSA-N | 363-24-6 |
| PGD2, Prostaglandin D₂ | 9α,15S-dihydroxy-11-oxo-prosta-5Z,13E-dien-1-oic acid | BHMBVRSPMRCCGG-OUTUXVNYSA-N | *41598-07-6* |
| PGF2, Prostaglandin F_{2α} | 9α,11α,15S-trihydroxy-prosta-5Z,13E-dien-1-oic acid | PXGPLTODNUVGFL-YNNPMVKQSA-N | *551-11-1* |
| TXB2, Thromboxane B₂ | 9α,11,15S-trihydroxythromba-5Z,13E-dien-1-oic acid | XNRNNGPBEPRNAR-JQBLCGNGSA-N | 54397-85-2 |
| 6-keto-PGF_{iα} | see above (Table 1) | see above (Table 1) | see above (Table 1) |

| **• 2b: LOX-metabolites** | | | |
|---|---|---|---|
| **Abbreviated name** | **Formal name** | **InChIKey** | **Cas-number** |
| LTB₄, Leukotriene B₄ | 5S,12R-dihydroxy-6Z,8E,10E,14Z-eicosatetraenoic acid | VNYSSYRCGWBHLG-AMOLWHMGSA-N | *71160-24-2* |
| 12-HETE | see above (Table 1) | see above (Table 1) | see above (Table 1) |
| 15-HETE | see above (Table 1) | see above (Table 1) | see above (Table 1) |
| 20-HETE | 20-hydroxy-5Z,8Z,11Z,14Z-eicosatetraenoic acid | NNDIXBJHNLFJJP-DTLRTWKJSA-N | *9551-86-3* |
| Hepoxilin A3 | (5Z,9E,14Z)-(11S,12S)-11,12-Epoxy-8-hydroxyicosa-5,9,14-trienoic acid | SGTUOBURCVMACZ-CIQDQOFUSA-N | 85589-24-8 |

| **• 2c: CYP-metabolites** | | | |
|---|---|---|---|
| **Abbreviated name** | **Formal name** | **InChIKey** | **Cas-number** |
| 5,6-EET | (±)5(6)-epoxy-8Z,11Z,14Z-eicosatrienoic acid | VBQNSZQZRAGRIX-GSKBNKFLSA-N | *87173-80-6* |
| 5,6-DHET | (±)5,6-dihydroxy-8Z,11Z,14Z-eicosatrienoic acid | GFNYAPAJUNPMGH-GSKBNKFLSA-N | *213382-49-1* |
| 8,9-EET | (±)8(9)-epoxy-5Z,11Z,14Z-eicosatrienoic acid) | DBWQSCSXHFNTMO-ZZMPYBMWSA-N) | 81246-85-7 |
| 8,9-DHET | (±)8,9-dihydroxy-5Z,11Z,14Z-eicosatrienoic acid | DCJBINATHQHPKO-ZZMPYBMWSA-N | *192461-96-4* |
| 11,12-EET | (±)11(12)-epoxy-5Z,8Z,14Z-eicosatrienoic acid | DXOYQVHGIODESM-LZXKBWHHSA-N | *123931-40-8* |
| 11,12-DHET | (±)11,12-dihydroxy-5Z,8Z,14Z-eicosatrienoic acid | LRPPQRCHCPFBPE-LZXKBWHHSA-N | 192461-95-3 |
| 14,15-EET | (±)14(15)-epoxy-5Z,8Z,11Z-eicosatrienoic acid | JBSCUHKPLGKXKH-KZTFMOQPSA-N | 81276-03-1 |
| 14,15-DHET | (±)14,15-dihydroxy-5Z,8Z,11Z-eicosatrienoic acid | SYAWGTIVOGUZMM-KZTFMOQPSA-N | 77667-09-5 |
| 9,10-EpOME | see above (Table 1) | see above (Table 1) | see above (Table 1) |
| 9,10-DiHOME | see above (Table 1) | see above (Table 1) | see above (Table 1) |
| 12,13-EpOME | (±)12(13)epoxy-9Z-octadecenoic acid | CCPPLLJZDQAOHD-GJGKEFFFSA-N | *503-07-1* |
| 12,13-DiHOME | 12,13-dihydroxy-9Z-octadecenoic acid | CQSLTKIXAJTQGA-GJGKEFFFSA-N | *263399-35-5* |
| 17,18-EEQ | (±)17(18)-epoxy-5Z,8Z,11Z,14Z-eicosatetraenoic acid | GPQW JQEBXAKBJ-YQLHGUCYSA-N | ?? |
| 19,20-EDP | (±)19(20)-epoxy-4Z,7Z,10Z,13Z,16Z-docosapentaenoic acid | OSXOPUBJJDUAOJ-MWEXLPNRSA-N | ?? |

### Terminology:

- **Oxidized lipid:** A lipid (fatty acid) that has been oxidized by an oxygenase enzyme (COX, cyclooxygenase; LOX, lipoxygenase or CYP, Cytochrome-P₄₅₀-Epoxygenase). These oxidized lipids have signaling functions in cells and mediate many different biological functions. The oxidization usually consists of the addition of a reactive group to the molecule (usually hydroxide or epoxide group → **epoxylipid).**
- **Peripheral neuropathic pain:** A disturbance of function or pathological change in a sensory nerve causing pain. This is mediated by a lesion or disease of the peripheral somatosensory nervous system and usually appears as pain in the extremities (feet or hands) caused by light mechanical stimulations (such as touch) or cold temperatures. Peripheral neuropathic pain, may as well appear without stimulations (spontaneous pain).
- **Chemotherapy-induced neuropathic pain:** Neuropathic pain that is a side effect (adverse event) of a cancer therapy and is caused by the toxicity of cancer therapeutics.
- **Cytostatics:** pharmacological substances for the treatment of cancer, such as **paclitaxel,** or **oxaliplatin.**
- **Amitriptyline, gabapentin and duloxetine:** Drugs that are approved for the treatment of neuropathic pain
- **LC-MS/MS: l**iquid **c**hromatography-tandem **m**ass **s**pectrometry, a coupled analytical method for the specific determination and quantification of low molecular weight analytes in biological samples.
- **InChIKey:** International Chemical Identifier for chemical substances that has been employed by the IUPAC (International Union of Pure and Applied Chemistry) for the specific identification of chemical subtances.

### Figures:

Figure 1: (A) Concentrations of 9,10-EpOME in nervous tissue (sciatic nerve, lumbar dorsal root ganglia (DRGs) and dorsal horn of the spinal cord 24h after i.p.-injection of paclitaxel (6 mg/kg) or oxaliplatin (3mg/kg). Data are shown as mean ± SEM from five mice per group; one-way ANOVA, *p<0.05, ***p<0.001. (B) time-course of mechanical allodynia after paclitaxel-injection in mice.
Figure 2: (A) Concentrations of 9,10-EpOME in nervous tissue (sciatic nerve, lumbar dorsal root ganglia (DRGs) and dorsal horn of the spinal cord 8d after multiple i.p.-injection of paclitaxel (4 x 2 mg/kg, injection every other day). (B) Concentrations of 6-keto-PGF_{1α} in nervous tissue (sciatic nerve, lumbar dorsal root ganglia and dorsal horn of the spinal cord 8d after multiple i.p.-injection of paclitaxel (4 x 2 mg/kg, injection every other day). Concentrations of 12S- (C) and 15S-HETE (D) 8d after i.p.-injection of paclitaxel (6 mg/kg) in nervous tissue. Data are shown as mean ± SEM from five mice per group; one-way ANOVA, *p<0.05, **p<0.01, n.d: not determined.
Figure 3: Concentrations of 13-HODE (A)and 9-HODE (B) in nervous tissue (sciatic nerve, lumbar dorsal root ganglia (DRGs) and dorsal horn of the spinal cord 10d after i.p.-injection of oxaliplatin (3 mg/kg, injection every other day). Data are shown as mean ± SEM from five mice per group; one-way ANOVA, *p<0.05.
Figure 4: Plasma-concentrations of 9,10-EpoME and its Metabolite 9,10-DiHOME 8d after paclitaxel-treatment (6 mg/kg) can be reduced by administration of telmisartan (10 mg/kg, 2h). Data are shown as mean ± SEM from five mice per group; one-way ANOVA, *p<0.05.

### EXAMPLES

### Materials and Methods

### Animals

All animal experiments were performed according to the recommendations in the Guide for the Care and Use of Laboratory Animals of the National Institutes of Health and approved by the local Ethics Committees for Animal Research (Darmstadt) with the permit number F95/42. For all behavioral experiments we used only 6-12 weeks old male C57BL/6N mice purchased from commercial breeding companies (Charles River, Sulzfeld, Germany, Janvier, Le Geneset-Saint-Isle, FR). To compare mechanical thresholds we used age and sex matched littermates as control.

### Models of chemotherapy-induced peripheral neuropathic pain

Paclitaxel was dissolved in Cremophor EL/Ethanol 1:1 and diluted in saline. The dose for intraperitoneal injection was set to 6 mg/kg as described previously [4]. Oxaliplatin was dissolved in saline. The dose for intraperitoneal injection was set to 3 mg/kg as described previously [5].

### Measurement of oxidized lipids from plasma using LC-MS/MS

### Standards and internal standards

For the measurement of epoxy lipids and HETEs stock solutions of the analytes 9,10-EpOME, 9-HODE, 13-HODE, 12-HETE und 15-HETE and internal standards 9,10-EpOME-d4, 9-HODE-d4, 13-HODE-d4, 12-HETE-d4 and 15-HETE-d4 are generated with concentrations of 2500 ng/ml in ethanol. Working standards were obtained by further dilution with a concentration range of 0.1-250 ng/ml for all analytes.

For prostanoids, stock solutions with 50,000 ng/ml of all analytes (6-keto-PGF_{1α}) and internal standards (6-keto-PGF_{1α}-d4) were prepared in methanol. Working standards were obtained by further dilution with a concentration range of 0.1-1,250 ng/ml

### Lipid-Extraction from Plasma

Lipids are extracted twice with 600 µl of ethyl acetate using liquid-liquid extraction. The combined organic phases were removed at a temperature of 45°C under a gentle stream of nitrogen. The residues were reconstituted with 50 µl of methanol/water/butylated hydroxytoluene (BHT) (50:50:10⁻³, v/v/v) (EpOMEs, HODEs and HETEs), or 50 µl of acetonitrile/water/formic acid (20:80:0.0025, v/v/v) (6-keto-PGF_{1α}) and then centrifuged for 2 min at 10,000 g, and transferred to glass vials waiting for analysis.

### Instrumentation for lipid measurement

The LC-MS/MS system consists of a QTrap 5500 (AB Sciex, Darmstadt, Germany) equipped with a Turbo-V source operating in negative electrospray ionization mode, an Agilent 1200 binary HPLC pump and degasser (Agilent, Waldbronn, Germany), and an HTC Pal autosampler (CTC analytics, Zwingen, Switzerland). High-purity nitrogen for the mass spectrometer was produced by a NGM 22-LC-MS nitrogen generator (cmc Instruments, Eschborn, Germany).

For the chromatographic separation of EpOMEs, HODEs and HETEs, a Gemini NX C18 column and precolumn were used (150 x 2 mm inner diameter, 5 µm particle size, and 110 Å pore size; Phenomenex, Aschaffenburg, Germany). For the chromatographic separation of prostanoids, a Synergi 4u Hydro-RP column (150 x 2 mm inner diameter, 4 µm, Phenomenex, Aschaffenburg, Germany) and a precolumn of same material were used.

### LC-Gradient and data analysis

For measurements of EpOMEs, HODEs und HETEs a linear gradient was used at a flow rate of 0.5 ml/min with a total run time of 17.5 min. Mobile phase A consist of water: ammonia (100:0.05, v/v), and mobile phase B of acetonitrile ammonia (100:0.05, v/v). The gradient changed from 85% A to 10% within 12 min. These conditions were held for 1 min. Then, the mobile phase shifted back to 85% A within 0.5 min and it was maintained for 4 min to re-equilibrate the column.

For the chromatographic separation of prostanoids, a Synergi 4u Hydro-RP column (150 x 2 mm inner diameter, 4 µm, Phenomenex, Aschaffenburg, Germany) and a precolumn of same material were used. Chromatographic separation was carried out in gradient elution mode at a flow rate of 0.3 ml/min. Total run time was 16 min. Mobile phase A consisted of water/formic acid (100:0.0025, v/v), and mobile phase B of acetonitrile/ formic acid (100:0.0025, v/v). The linear gradient started with 90% A for 1 min and then changed to 60% A within 1 min. It was held for 1 min at 60% in phase A. Within 1min, the mobile phase shifted to 50% in phase A and was held for 2 min. Within 2 min, the mobile phase shifted to 10% A and was held for 1 min. Composition of the gradient shifted back to 90% A in one min and it was maintained for 6 min to re-equilibrate the column.

A volume of 20 µl (EpOMEs, HODEs und HETEs) or 45 µl (prostanoids) of the extracted samples was injected into the LC-MS/MS system. Quantification was performed with Analyst software version 1.6 (Applied Biosystems) using the internal standard method (isotope-dilution mass spectrometry). Ratios of analyte peak area and internal standard area (y-axis) were plotted against concentration (x-axis), and calibration curves were calculated by least-squares regression with 1/square concentration weighting.

### References

1. Finnerup NB, Attal N, Haroutounian S, McNicol E, Baron R, Dworkin RH, Gilron I, Haanpaa M, Hansson P, Jensen TS et al: Pharmacotherapy for neuropathic pain in adults: a systematic review and meta-analysis. Lancet Neurol 2015, 14(2):162-173.
2. Dworkin RH, O'Connor AB, Audette J, Baron R, Gourlay GK, Haanpaa ML, Kent JL, Krane EJ, Lebel AA, Levy RM et al: Recommendations for the pharmacological management of neuropathic pain: an overview and literature update. Mayo Clin Proc 2010, 85(3 Suppl):S3-14.
3. Borsook D, Becerra L, Hargreaves R: Biomarkers for chronic pain and analgesia. Part 1: the need, reality, challenges, and solutions. Discov Med 2011, 11(58):197-207.
4. Alessandri-Haber N, Dina OA, Joseph EK, Reichling DB, Levine JD: Interaction of transient receptor potential vanilloid 4, integrin, and SRC tyrosine kinase in mechanical hyperalgesia. J Neurosci 2008, 28(5):1046-1057.
5. Nassini R, Gees M, Harrison S, De Siena G, Materazzi S, Moretto N, Failli P, Preti D, Marchetti N, Cavazzini A et al: Oxaliplatin elicits mechanical and cold allodynia in rodents via TRPA1 receptor stimulation. Pain 2011, 152(7):1621-1631.
6. Materazzi S, Fusi C, Benemei S, Pedretti P, Patacchini R, Nilius B, Prenen J, Creminon C, Geppetti P, Nassini R: TRPA1 and TRPV4 mediate paclitaxel-induced peripheral neuropathy in mice via a glutathione-sensitive mechanism. Pflugers Arch 2012, 463(4):561-569.
7. Bennett DL, Woods CG: Painful and painless channelopathies. Lancet Neurol 2014.
8. Kremeyer B, Lopera F, Cox JJ, Momin A, Rugiero F, Marsh S, Woods CG, Jones NG, Paterson KJ, Fricker FR et al: A gain-of-function mutation in TRPA1 causes familial episodic pain syndrome. Neuron 2010, 66(5):671-680
9. Poulsen J N et al; "Ibudilast and SKF modulate cisplatin-evoked PGE2 release from isoalted trigeminal satellite glial cells" GLIA, vol. 61, Suppl 1; July 2013, pg S109; 11 th European Meeting on Glial cell function in health and disease, Berlin, Germany July 3-6, 2013.

## Claims

1. A method for the prediction of the onset or duration of chronic or neuropathic pain in a subject, comprising:
a) separating plasma from blood sample;
b) measuring the concentration of an oxidized lipid in the plasma;
c) determining the ratio of the elevation of the oxidized lipid;
wherein said oxidized lipid is selected from the group consisting of: 9,10-EpOME ((±)9(10)-epoxy-12Z-octadecaenoic acid), 9-HODE ((±)9-hydroxy-10(E),12(Z)-octadecadienoic acid) and 13-HODE ((±)13-hydroxy-9(Z),11(E)-octadecadienoic acid), wherein said chronic or neuropathic pain is peripheral neuropathy due to chemotherapy, and wherein the concentration of the lipid is measured after 24 hours after the start of chemotherapy.

2. Method according to claim 1, wherein said lipid is 9,10-EpOME ((±)9(10)-epoxy-12Z-octadecaenoic acid.

3. Method according to claim 1 or 2, wherein the concentration is measured from plasma.

4. Method according to claims 1 to 3, wherein the concentration is measured using LC-MS/MS.

5. A cytochrome P450 expoxygenase (CYP)-antagonist for use in the treatment of peripheral neuropathy due to chemotherapy in a subject, **characterized in that** the therapy starts before the first symptoms arise in a patient, after the elevation of an oxidized lipid has been detected, wherein said lipid is selected from the group consisting of: 9,10-EpOME ((±)9(10)-epoxy-12Z-octadecaenoic acid), 9-HODE ((±)9-hydroxy-10(E),12(Z)-octadecadienoic acid) and 13-HODE ((±)13-hydroxy-9(Z),11(E)-octadecadienoic acid), wherein the treatment of the pain in a subject starts when the concentration of the lipid is at least 20% higher than the normal value, and wherein the concentration of the lipid is measured after 24 hours after the start of chemotherapy.

6. Cytochrome P450 expoxygenase (CYP)-antagonist for use in the treatment of peripheral neuropathy due to chemotherapy in a subject according to claim 5, wherein the treatment of the pain in a subject starts when the concentration of the lipid is at least 30% higher than the normal value.

7. Cytochrome P450 expoxygenase (CYP)-antagonist for use in the treatment of peripheral neuropathy due to chemotherapy in a subject according to claim 5 or 6, wherein the treatment of the pain in a subject starts when the concentration of the lipid is at least 40% higher than the normal value.

8. Cytochrome P450 expoxygenase (CYP)-antagonist for use in the treatment peripheral neuropathy due to chemotherapy in a subject according to claims 5 to 7, wherein the CYP-antagonist is selected from the group consisting of a CYP1A-, CYP2B-, CYP2C-, CYP2E-, and a CYP2J-antagonist.

9. Cytochrome P450 expoxygenase (CYP)-antagonist for use in the treatment of peripheral neuropathy due to chemotherapy in a subject according to claim 8, wherein the CYP-antagonist is an antagonist of a mammalian homologue of CYP2J2 (CYP2J2-antagonist), such as telmisartan, aripiprazole or most preferably terfenadine.

10. Cytochrome P450 expoxygenase (CYP)-antagonist for use in the treatment of peripheral neuropathy due to chemotherapy in a subject according to claims 5 to 9, wherein the therapy starts after the elevation of an oxidized lipid has been detected according to the methods as claimed in claims 1 to 4.

## Patentansprüche

1. Verfahren zur Vorhersage des Beginns bzw. der Dauer chronischer oder neuropathischer Schmerzen bei einem Individuum, umfassend:
a) Trennen von Plasma von einer Blutprobe;
b) Messen der Konzentration eines oxidierten Lipids im Plasma;
c) Bestimmen des Verhältnisses der Erhöhung des oxidierten Lipids;
wobei das oxidierte Lipid ausgewählt ist aus der Gruppe bestehend aus: 9,10-EpOME ((±)9(10)-Epoxy-12Z-octadecensäure), 9-HODE ((±)9-Hydroxy-10(E),12(Z)-octadecadiensäure) und 13-HODE ((±)13-Hydroxy-9(Z),11(E)-octadecadiensäure), wobei es sich bei den chronischen oder neuropathischen Schmerzen um periphere Neuropathie aufgrund einer Chemotherapie handelt und wobei die Konzentration des Lipids 24 Stunden nach dem Beginn der Chemotherapie gemessen wird.

2. Verfahren nach Anspruch 1, wobei es sich bei dem Lipid um 9,10-EpOME ((±)9(10)-Epoxy-12Z-octadecensäure) handelt.

3. Verfahren nach Anspruch 1 oder 2, wobei die Konzentration aus Plasma gemessen wird.

4. Verfahren nach Anspruch 1 bis 3, wobei die Konzentration unter Verwendung von LC-MS/MS gemessen wird.

5. Cytochrom-P450-Expoxygenase(CYP)-Antagonist zur Verwendung bei der Behandlung von peripherer Neuropathie aufgrund einer Chemotherapie bei einem Individuum, **dadurch gekennzeichnet, dass** mit der Therapie begonnen wird, bevor die ersten Symptome bei einem Patienten auftreten, nachdem die Erhöhung eines oxidierten Lipids nachgewiesen wurde, wobei das Lipid ausgewählt ist aus der Gruppe bestehend aus: 9,10-EpOME ((±)9(10)-Epoxy-12Z-octadecen-säure), 9-HODE (±)9-Hydroxy-10(E),12(Z)-octa-decadiensäure) und 13- HODE ((±)13-Hydroxy-9(Z),11(E)-octadecadiensäure), wobei die Behandlung der Schmerzen bei einem Individuum beginnt, wenn die Konzentration des Lipids wenigstens 20% höher als der Normalwert ist, und wobei die Konzentration des Lipids 24 Stunden nach dem Beginn der Chemotherapie gemessen wird.

6. Cytochrom-P450-Expoxygenase(CYP)-Antagonist zur Verwendung bei der Behandlung von peripherer Neuropathie aufgrund einer Chemotherapie bei einem Individuum nach Anspruch 5, wobei die Behandlung der Schmerzen bei einem Individuum beginnt, wenn die Konzentration des Lipids wenigstens 30% höher als der Normalwert ist.

7. Cytochrom-P450-Expoxygenase(CYP)-Antagonist zur Verwendung bei der Behandlung von peripherer Neuropathie aufgrund einer Chemotherapie bei einem Individuum nach Anspruch 5 oder 6, wobei die Behandlung der Schmerzen bei einem Individuum beginnt, wenn die Konzentration des Lipids wenigstens 40% höher als der Normalwert ist.

8. Cytochrom-P450-Expoxygenase(CYP)-Antagonist zur Verwendung bei der Behandlung peripherer Neuropathie aufgrund einer Chemotherapie bei einem Individuum nach den Ansprüchen 5 bis 7, wobei der CYP-Antagonist ausgewählt ist aus der Gruppe bestehend aus einem CYP1A-, CYP2B-, CYP2C-, CYP2E- und einem CYP2J-Antagonisten.

9. Cytochrom-P450-Expoxygenase(CYP)-Antagonist zur Verwendung bei der Behandlung von peripherer Neuropathie aufgrund einer Chemotherapie bei einem Individuum nach Anspruch 8, wobei es sich bei dem CYP-Antagonisten um einen Antagonisten eines Säugerhomologs von CYP2J2 (CYP2J2-Antagonist), wie z. B. Telmisartan, Aripiprazol oder ganz besonders bevorzugt Terfenadin, handelt.

10. Cytochrom-P450-Expoxygenase(CYP)-Antagonist zur Verwendung bei der Behandlung von peripherer Neuropathie aufgrund einer Chemotherapie bei einem Individuum nach den Ansprüchen 5 bis 9, wobei mit der Therapie begonnen wird, nachdem die Erhöhung eines oxidierten Lipids nach den Verfahren gemäß den Ansprüchen 1 bis 4 nachgewiesen wurde.

## Revendications

1. Procédé pour la prédiction de l'apparition ou de la durée d'une douleur chronique ou neuropathique chez un sujet, comprenant :
a) la séparation du plasma d'un échantillon sanguin ;
b) la mesure de la concentration d'un lipide oxydé dans le plasma ;
c) la détermination du taux d'augmentation du lipide oxydé ;
dans lequel ledit lipide oxydé est choisi dans le groupe consistant en : le 9,10-EpOME (acide (±)9(10)-époxy-12Z-octadécaénoïque), le 9-HODE (acide (±9-hydroxy-10(E),12(Z)-octadécadiénoïque) et le 13-HODE (acide (±13-hydroxy-9(Z),11(E)-octadécadiénoïque), dans lequel ladite douleur chronique ou neuropathique est une neuropathie périphérique due à une chimiothérapie, et dans lequel la concentration du lipide est mesurée après 24 heures après le début de la chimiothérapie.

2. Procédé selon la revendication 1, dans lequel ledit lipide est le 9,10-EpOME (acide (±)9(10)-époxy-12Z-octadécaénoïque).

3. Procédé selon la revendication 1 ou 2, dans lequel la concentration est mesurée dans le plasma.

4. Procédé selon les revendications 1 à 3, dans lequel la concentration est mesurée par utilisation d'une chromatographie en phase liquide avec spectrométrie de masse en tandem, LC-MS/MS.

5. Antagoniste de la cytochrome P450 époxygénase (CYP) pour une utilisation dans le traitement d'une neuropathie périphérique due à une chimiothérapie chez un sujet, **caractérisé en ce que** la thérapie commence avant que les premiers symptômes n'apparaissent chez un patient, après que l'élévation d'un lipide oxydé a été détectée, dans lequel ledit lipide est choisi dans le groupe consistant en : le 9,10-EpOME (acide (±)9(10)-époxy-12Z-octadécaénoïque), le 9-HODE (acide (±9-hydroxy-10(E),12(Z)-octadécaénoïque) et le 13-HODE (acide (±13-hydroxy-9(Z),11(E)-octadécaénoïque), dans lequel le traitement de la douleur chez un sujet commence lorsque la concentration du lipide est d'au moins 20 % supérieure à la valeur normale, et dans lequel la concentration du lipide est mesurée après 24 heures après le début de la chimiothérapie.

6. Antagoniste de la cytochrome P450 époxygénase (CYP) pour une utilisation dans le traitement d'une neuropathie périphérique due à une chimiothérapie chez un sujet selon la revendication 5, dans lequel le traitement de la douleur chez un sujet commence lorsque la concentration du lipide est d'au moins 30 % supérieure à la valeur normale.

7. Antagoniste de la cytochrome P450 époxygénase (CYP) pour une utilisation dans le traitement d'une neuropathie périphérique due à une chimiothérapie chez un sujet selon la revendication 5 ou 6, dans lequel le traitement de la douleur chez un sujet commence lorsque la concentration du lipide est d'au moins 40 % supérieure à la valeur normale.

8. Antagoniste de la cytochrome P450 époxygénase (CYP) pour une utilisation dans le traitement d'une neuropathie périphérique due à une chimiothérapie chez un sujet selon les revendications 5 à 7, dans lequel l'antagoniste de la CYP est choisi dans le groupe consistant en un antagoniste de la CYP1A, de la CYP2B, de la CYP2C, de la CYP2E et de la CYP2J.

9. Antagoniste de la cytochrome P450 époxygénase (CYP) pour une utilisation dans le traitement d'une neuropathie périphérique due à une chimiothérapie chez un sujet selon la revendication 8, dans lequel l'antagoniste de la CYP est un antagoniste d'un homologue de mammifère de la CYP2J2 (antagoniste de la CYP2J2), tel que le telmisartan, l'aripiprazole ou plus préférablement la terfénadine.

10. Antagoniste de la cytochrome P450 époxygénase (CYP) pour une utilisation dans le traitement d'une neuropathie périphérique due à une chimiothérapie chez un sujet selon les revendications 5 à 9, dans lequel la thérapie commence après que l'augmentation d'un lipide oxydé a été détectée par les procédés selon les revendications 1 à 4.
